# EUROPEAN PATENT APPLICATION

(11) **EP 3 878 873 A1**
(43) Date of publication of application: **15.09.2021**
(21) Application number: 19908953.3
(22) Date of filing: 12.12.2019
(51) Int. Cl.: C08F 8/44, A61L 27/52, A61L 31/14, A61C 8/02, A61B 90/00, C08J 3/075

(54) **MODIFIED HYDROGEL AND GINGIVA EXTENSION DEVICE COMPRISING SAME**

(30) Priority: 09.01.2019 KR 20190002534
(71) Applicant: Osstemimplant Co., Ltd., Seoul 08505 (KR)
(72) Inventor: KIM, Min Kyoung, Busan 47206 (KR); JANG, Il-Seok, Yangsan-si, Gyeongsangnam-do 50653 (KR); SONG, Ju Dong, Busan 48272 (KR)
(74) Representative: Isarpatent
(86) International application number: PCT/KR2019/017578
(87) International publication number: WO 2020/145529

(57) **Abstract**

One aspect of the present invention provides a modified hydrogel and a gingival extension device comprising same, the modified hydrogel being characterized in that at least a part of a carboxyl group (-COOH) located in a side chain of a polymer constituting the hydrogel is modified and represented by the formula as follows: <formula> P-(COO⁻)ₙ Aⁿ⁺ wherein, P is the backbone of the polymer, n is one integer of 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and two or more combinations thereof.

## Description

### [Technical Field]

The present invention relates to a modified hydrogel and a gingival extension device including the same, and more specifically, to a modified hydrogel capable of precisely controlling an expansion rate and a gingival extension device including the same.

### [Background Art]

An endosseous implant in which the implant is placed inside the jaw bone may require a suitable amount of bone around an implant fixture as a prerequisite for successful osseointegration and initial stability for a successful surgery.

When the suitable amount or volume of bone for implant placement is not satisfied, or when a part of the fixture is exposed to the outside of the bone for various reasons (such as alveolar bone resorption due to inflammation) after implant placement, guided bone regeneration-augmentation (GBR) is used.

The guided bone regeneration-augmentation is a surgery intended to regenerate bone tissues by forming a space under a barrier membrane using the barrier membrane when there is an insufficient amount of bone around an implanted implant or at a position where the implant will be implanted, and inducing the influx of osteoblasts into a bone defective part in a lower part of the space. In the guided bone regeneration-augmentation, various bone materials (autogenous bone, allogeneic bone, heterogenous bone, synthetic bone) are used together with resorbable and non-resorbable dental barrier membranes.

The dental barrier membrane prevents the gingival epithelium, which has excellent regenerative power, from growing and entering a damaged part first, and has the ability to retain space, and thus assists connective tissue differentiation and bone regeneration.

Meanwhile, in the implantation of an implant, when the amount of bone is insufficient, it may be difficult to secure a space for alveolar bone regeneration due to gingival recession.

To solve these problems, a hydrogel-based gingival extension device can be implanted prior to guided bone regeneration-augmentation. The gingival extension device can be used to reconstruct a receded gingiva into a normal state due to the swelling nature thereof.

Existing gingival extension devices have been developed based on a hydrogel based on a copolymer of methyl methacrylate (MMA) and N-vinyl-2-pyrrolidone (VP), but the focus was only on the space-securing function by simple swelling.

In general, the hydrogel is sealed with a silicone membrane in which a plurality of through holes are formed and then applied to a gingival extension device. The size and number of the plurality of through holes formed in the silicone membrane directly affects the expansion rate of the hydrogel by adjusting the inflow rate and amount of body fluid. However, there are problems in that it is difficult to finely adjust the size of the through holes, and durability deteriorates due to the reduction in mechanical properties of the silicon membrane, particularly, tensile strength, as the number of holes is increased in order to adjust the expansion rate.

Therefore, there is a need for developing a technique capable of easily adjusting the expansion rate of a hydrogel while maintaining the mechanical properties of a silicon membrane, without depending on the size and number of through holes of the silicon membrane as in the related art.

### [Disclosure]

### [Technical Problem]

The present invention has been made in an effort to solve the above-described problems in the related art, and an object of the present invention is to provide a modified hydrogel capable of easily adjusting the expansion rate of the hydrogel while maintaining the mechanical properties of a silicon membrane, without depending on the size and number of through holes of the silicon membrane, and a gingival extension device including the same.

### [Technical Solution]

One aspect of the present invention provides a modified hydrogel in which at least a part of a carboxyl group (-COOH) located in a side chain of a polymer constituting the hydrogel is modified and represented by the formula as follows.

<Formula> P-(COO⁻)ₙAⁿ⁺

wherein P is the backbone of the polymer, n is an integer ranging from 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and combinations of two or more thereof.

In an embodiment, the polymer may be a polymer in which a first monomer having a methacrylate and a second monomer having a vinyl group are cross-linked.

In an embodiment, the first monomer may be methyl methacrylate.

In an embodiment, the second monomer may be N-vinyl-2-pyrrolidone.

Another aspect of the present invention provides a gingival extension device including: the modified hydrogel; and a porous membrane which seals the modified hydrogel.

In an embodiment, the porous membrane may be composed of silicone.

Still another aspect of the present invention provides a method for preparing a modified hydrogel, the method including: (a) a step of preparing a hydrogel polymer having at least one carboxyl group (-COOH) in a side chain of the polymer by polymerizing a first monomer having a methacrylate and a second monomer having a vinyl group; (b) a step of preparing a solution by dissolving an ionic compound; and (c) a step of modifying at least a part of the carboxyl group (-COOH) into a structure represented by the following formula by immersing the hydrogel polymer in the solution.

<Formula> P-(COO⁻)ₙAⁿ⁺

wherein P is the backbone of the polymer, n is an integer ranging from 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and combinations of two or more thereof.

In an embodiment, the first monomer may be methyl methacrylate.

In an embodiment, the second monomer may be N-vinyl-2-pyrrolidone.

In an embodiment, a content of the ionic compound in the solution may be 3 to 25 wt%.

In an embodiment, a pH of the solution may be 5 to 8.5.

### [Advantageous Effects]

In the modified hydrogel according to one aspect of the present invention, a proton (H⁺) of a carboxyl group (-COOH) located in a side chain of a polymer is substituted with a predetermined cation by ion exchange, so that the expansion rate of the hydrogel can be easily adjusted by adjusting the dissociation rate of the cation according to the bonding strength between the substituted cation and the -COO⁻, which can be implemented even in a state in which the mechanical properties of a silicone membrane are not inhibited, without depending on the size and number of through holes of a silicone membrane in the related art.

The effects of the present invention are not limited to the aforementioned effects and should be understood to include all possible effects deduced from the configuration of the invention described in the detailed description or the claims of the present invention.

### [Description of Drawings]

FIG. 1 illustrates the expansion mechanism of a modified hydrogel according to embodiments of the present invention.
FIGS. 2 and 3 are graphs showing the expansion rates of the hydrogel molded article according to the examples and comparative examples of the present invention.

### [Modes of the Invention]

Hereinafter, the present invention will be described with reference to the accompanying drawings. However, the present invention can be realized in various different forms, and is not limited to the embodiments described herein. In addition, in order to clearly describe the present invention, portions that are not related to the description are omitted in the drawings, and like reference numerals are added to like portions throughout the specification.

Throughout the specification, when one part is "connected" to another part, this includes not only a case where they are "directly connected to each other", but also a case where they are "indirectly connected to each other" with another member interposed therebetween. Further, when one part "includes" one constituent element, unless otherwise specifically described, this does not mean that another constituent element is excluded, but means that another constituent element may be further included.

As used herein, the "hydrogel" refers to a gel using water as a dispersion medium, and the hydrogel may lose its fluidity due to cooling, or may be formed by allowing a hydrophilic polymer having a three-dimensional network structure and a microcrystalline structure to contain water and be expanded.

### Modified hydrogel

One aspect of the present invention provides a modified hydrogel in which at least a part of a carboxyl group (-COOH) located in a side chain of a polymer constituting the hydrogel is modified and represented by the formula as follows.

<Formula> P-(COO⁻)ₙAⁿ⁺

wherein, P is the backbone of the polymer, n is an integer ranging from 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and combinations of two or more thereof.

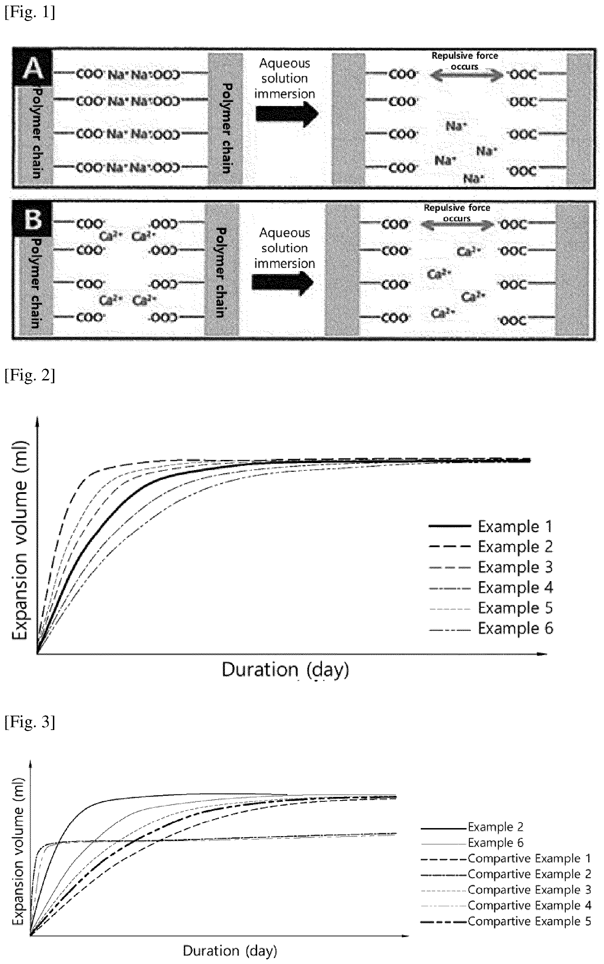

FIG. 1 illustrates the expansion mechanism of a modified hydrogel according to embodiments of the present invention. Referring to FIG. 1, Aⁿ⁺ may be a monovalent or polyvalent cation, and the larger n is, the stronger the bond with - COO⁻ is, so that the dissociation rate from the polymer and the accompanying expansion rate become slower. In contrast, the smaller n is, the weaker the bond with -COO⁻ is, so that the dissociation rate from the polymer and the accompanying expansion rate become faster. Therefore, the modified hydrogel may be designed according to the expansion rate, and when the designed modified hydrogel is commercialized as a gingival extension device, a practitioner may selectively and variably apply a gingival extension device controlled at a constant expansion rate according to the condition of an affected area.

The hydrogel serves to secure the gingival space through swelling in the body by absorbing body fluid, and may be preferably a hydrogel in which a first monomer having a methacrylate and a second monomer having a vinyl group are cross-linked. For example, the hydrogel may be synthesized by cross-linking methyl methacrylate (MMA) and N-vinyl-2-pyrrolidone (VP) at a mass ratio of 6 to 7 : 4 to 3, and may also be synthesized by cross-linking hydroxyethyl methacrylate (HEMA) and N-vinyl-2-pyrrolidone (VP), or n-methacrylate (n=carbon number, 4, 8, 12) and N-vinyl-2-pyrrolidone (VP)(n=4: butyl methacrylate, n=8: octyl methacrylate, n=12: lauryl methacrylate).

When the body fluid is absorbed by the hydrogel, a -COOH group located in a side chain of a polymer constituting the hydrogel is dissociated into -COO⁻ and H⁺, and -COO⁻ increases the repulsive force between the hydrogel polymer backbones, and due to this, as the distance between the polymer backbones increases, there is a self-expanding characteristic. The hydrogel thus expanded may be expanded into a round cylinder shape capable of generally expanding the gingiva widely, regardless of the initial shape.

### Gingival extension device

Another aspect of the present invention provides a gingival extension device including: the modified hydrogel; and a porous membrane which seals the modified hydrogel. The porous membrane may be formed of silicone.

The porous membrane is a perforated or semi-permeable membrane, and may include through holes through which body fluids or fluids can permeate. Therefore, the diffusion rate of the body fluid inside the gingival extension device may be controlled by adjusting the size and number of through holes of the porous membrane.

The through holes may have a diameter of 0.3 to 0.7 mm In this case, the through holes may have a diameter of 0.3 mm or more, 0.35 mm or more, or 0.4 mm or more, and 0.7 mm or less, 0.65 mm or less, or 0.6 mm or less. When the through holes have a diameter less than 0.3 mm, the expansion rate of the hydrogel becomes excessively slow due to the small inflow of body fluid, and when the through holes have a diameter more than 7 mm, the inflow of body fluid is increased, causing rapid swelling of the hydrogel, which may adversely affect the restoration and healing of an affected area.

If necessary, one side or both sides of the porous membranes may further include a bonding portion bonded by an adhesive. The bonding portion is fixed to the alveolar bone using a screw. Preferably, both sides of the porous membrane may include a bonding portion, and the bonding portion may be perfectly fixed to the alveolar bone using a screw, thereby preventing unnecessary movement within the gingiva. Therefore, the tissue may be expanded into a desired site, and a gingival extension device within the gingiva does not move, and thus may also help restore neighboring soft tissue.

### Method for preparing modified hydrogel

Still another aspect of the present invention provides a method for preparing a modified hydrogel, the method including: (a) a step of preparing a hydrogel polymer having at least one carboxyl group (-COOH) in a side chain of the polymer by polymerizing a first monomer having a methacrylate and a second monomer having a vinyl group; (b) a step of preparing a solution by dissolving an ionic compound; and (c) a step of modifying at least a part of the carboxyl group (-COOH) into a structure represented by the following formula by immersing the hydrogel polymer in the solution.

<Formula> P-(COO⁻)ₙAⁿ⁺

wherein P is the backbone of the polymer, n is an integer ranging from 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and combinations of two or more thereof.

The effects of the cations introduced by the modification of the hydrogel polymer on the expansion mechanism of the hydrogel are as described above.

The hydrogel may be synthesized by cross-linking methyl methacrylate (MMA) and N-vinyl-2-pyrrolidone (VP) at a mass ratio of 6 to 7 : 4 to 3, and may also be synthesized by cross-linking hydroxyethyl methacrylate (HEMA) and N-vinyl-2-pyrrolidone (VP), or n-methacrylate (n=carbon number, 4, 8, 12) and N-vinyl-2-pyrrolidone (VP)(n=4: butyl methacrylate, n=8: octyl methacrylate, n=12: lauryl methacrylate).

The ionic compound may be, for example, an ionic bonding compound consisting of metal or non-metal cations and anions, and the type thereof is not particularly limited as long as it can be dissociated into cations and anions in a solution.

A content of the ionic compound in the solution may be 3 to 25 wt%. In this case, the content of the ionic compound may be 3 wt% or more, 5 wt% or more, or 7 wt% or more, and 25 wt% or less, 23 wt% or less, or 21 wt% or less. When the content of the ionic compound is less than 3 wt%, the effect of adjusting the expansion rate of the modified hydrogel may be weak, and when the content exceeds 25 wt%, the inherent expansion behavior of the hydrogel polymer is inhibited, so that it is difficult to expand to a required volume.

Further, a pH of the solution may be 5 to 8.5. In this case, the pH of the solution may be 5 or more, 5.5 or more, or 6 or more, and 8.5 or less, 8 or less, or 7.5 or less. When the pH of the solution is less than 5, the effect of adjusting the expansion rate of the modified hydrogel may be weak, and when the pH exceeds 8.5, the inherent expansion behavior of the hydrogel polymer is inhibited, so that it is difficult to expand to a required volume.

Hereinafter, the Examples of the present invention will be described in detail.

### Example 1

After methyl methacrylate (MMA), N-vinyl-2-pyrrolidone (VP), 0.1 wt% of 2,2'-azobisisobutyronitrile (an initiator), 0.2 wt% of hydroxypropyl methacrylate (a cross-linking agent), and methyl pyrrolidone (a solvent) were blended and mixed so as to have an entire solution concentration of 0.8 M, wherein the cross-linked methyl methacrylate (MMA) and N-vinyl-2-pyrrolidone (VP) had a mass ratio of 6 to 7 : 3 to 4, the mixture was thermally polymerized at a temperature of 65°C for 24 hours. Then, a hydrogel polymer in which methyl methacrylate (MMA) and N-vinyl-2-pyrrolidone (VP) were crosslinked was synthesized by removing a residue such as a solvent and performing hydrolysis.

A proton (H⁺) of a carboxyl group (-COOH) located in a side chain of the cross-linked hydrogel polymer was substituted with Na⁺ by ion exchange by immersing the hydrogel polymer in an aqueous NaCl solution having a concentration of 10 wt% and a pH of 6.5, and then a residue such as a solvent was removed, the resulting product was placed in a prismatic compression mold and compressed and dried for 5 hours by applying a pressure of 8 MPa on average and a temperature of 60°C on average, thereby obtaining a hydrogel molded article.

### Example 2

After methyl methacrylate (MMA), N-vinyl-2-pyrrolidone (VP), 0.1 wt% of 2,2'-azobisisobutyronitrile (an initiator), 0.2 wt% of hydroxypropyl methacrylate (a cross-linking agent), and methyl pyrrolidone (a solvent) were blended and mixed so as to have an entire solution concentration of 0.8 M, wherein the cross-linked methyl methacrylate (MMA) and N-vinyl-2-pyrrolidone (VP) had a mass ratio of 6 to 7 : 3 to 4, the mixture was thermally polymerized at a temperature of 65°C for 24 hours. Then, a hydrogel polymer in which methyl methacrylate (MMA) and N-vinyl-2-pyrrolidone (VP) were crosslinked was synthesized by removing a residue such as a solvent and hydrolyzing the resulting product.

A proton (H⁺) of a carboxyl group (-COOH) located in a side chain of the cross-linked hydrogel polymer was substituted with Ca²⁺ by ion exchange by immersing the hydrogel polymer in an aqueous CaCl₂ solution having a concentration of 10 wt% and a pH of 6.8, and then a residue such as a solvent was removed, the resulting product was placed in a prismatic compression mold and compressed and dried for 5 hours by applying a pressure of 8 MPa on average and a temperature of 60°C on average, thereby obtaining a hydrogel molded article.

### Example 3

A hydrogel molded article was obtained in the same manner as in Example 1, except that the concentration of the aqueous NaCl solution was changed to 25 wt%.

### Example 4

A hydrogel molded article was obtained in the same manner as in Example 1, except that the concentration of the aqueous NaCl solution was changed to 3 wt%.

### Example 5

A hydrogel molded article was obtained in the same manner as in Example 1, except that the pH of the aqueous NaCl solution was changed to 8.5.

### Example 6

A hydrogel molded article was obtained in the same manner as in Example 1, except that the pH of the aqueous NaCl solution was changed to 5.0.

### Comparative Example 1

After methyl methacrylate (MMA), N-vinyl-2-pyrrolidone (VP), 0.1 wt% of 2,2'-azobisisobutyronitrile (an initiator), 0.2 wt% of hydroxypropyl methacrylate (a cross-linking agent), and methyl pyrrolidone (a solvent) were blended and mixed so as to have an entire solution concentration of 0.8 M, wherein the cross-linked methyl methacrylate (MMA) and N-vinyl-2-pyrrolidone (VP) had a mass ratio of 6 to 7 : 3 to 4, the mixture was thermally polymerized at a temperature of 65°C for 24 hours. Then, a hydrogel gel in which methyl methacrylate (MMA) and N-vinyl-2-pyrrolidone (VP) were crosslinked was synthesized by removing a residue such as a solvent and performing hydrolysis.

The cross-linked hydrogel was placed in a prismatic compression mold and compressed and dried for 5 hours by applying a pressure of 8 MPa on average and a temperature of 60°C on average, thereby obtaining a hydrogel molded article.

### Comparative Example 2

A hydrogel molded article was obtained in the same manner as in Example 1, except that the concentration of the aqueous NaCl solution was changed to 30 wt%.

### Comparative Example 3

A hydrogel molded article was obtained in the same manner as in Example 1, except that the concentration of the aqueous NaCl solution was changed to 2.5 wt%.

### Comparative Example 4

A hydrogel molded article was obtained in the same manner as in Example 1, except that the pH of the aqueous NaCl solution was changed to 9.0.

### Comparative Example 5

A hydrogel molded article was obtained in the same manner as in Example 1, except that the pH of the aqueous NaCl solution was changed to 4.5.

### Experimental Example

The hydrogel molded articles according to Examples and Comparative Examples were immersed in physiological saline in an incubator at 37°C for 40 days and expanded, and the results are illustrated in FIGS. 2 and 3.

The above-described description of the present invention is provided for illustrative purposes, and a person skilled in the art to which the present invention pertains will understand that the present invention can be easily modified into other specific forms without changing the technical spirit or essential features of the present invention. Therefore, it should be understood that the above-described embodiments are only illustrative in all aspects and not restrictive. For example, each constituent element which is described as a singular form may be implemented in a distributed form, and similarly, constituent elements which are described as being distributed may be implemented in a combined form.

The scope of the present invention is represented by the claims to be described below, and it should be interpreted that the meaning and scope of the claims and all the changes or modified forms derived from the equivalent concepts thereto fall within the scope of the present invention.

## Claims

1. A modified hydrogel in which at least a part of a carboxyl group (-COOH) located in a side chain of a polymer constituting the hydrogel is modified and represented by the formula as follows,
<Formula> P-(COO⁻)ₙAⁿ⁺
wherein P is the backbone of the polymer, n is an integer ranging from 1 to 3, and A is one selected from the group consisting of Na, K, NH4, Ca, Ng, Zn, Fe, Al, Sn, Cu, Ni, Mn, W, Co, Ag, Pb, Au, Pd, Pt, Ti, and combinations of two or more thereof.

2. The modified hydrogel of claim 1, wherein the polymer is a polymer in which a first monomer having a methacrylate and a second monomer having a vinyl group are cross-linked.

3. A gingival extension device comprising: the modified hydrogel of claim 1 or 2; and a porous membrane which seals the modified hydrogel.

4. The gingival extension device of claim 3, wherein the porous membrane is formed of silicone.

5. A method for preparing a modified hydrogel, the method comprising:
(a) a step of preparing a hydrogel polymer having at least one carboxyl group (-COOH) in a side chain of the polymer by polymerizing a first monomer having a methacrylate and a second monomer having a vinyl group;
(b) a step of preparing a solution by dissolving an ionic compound; and
(c) a step of modifying at least a part of the carboxyl group (-COOH) into a structure represented by the following formula by immersing the hydrogel polymer in the solution.
<Formula> P-(COO⁻)ₙAⁿ⁺
wherein P is the backbone of the polymer, n is an integer ranging from 1 to 3, and A is one selected from the group consisting of Na, K, NH₄, Ca, Ng, Zn, Fe, Al, Sn, Cu, Ni, Mn, W, Co, Ag, Pb, Au, Pd, Pt, Ti, and combinations of two or more thereof.

6. The method of claim 5, wherein the first monomer is methyl methacrylate.

7. The method of claim 5, wherein the second monomer is N-vinyl-2-pyrrolidone.

8. The method of claim 5, wherein a content of the ionic compound in the solution is 3 to 25 wt%.

9. The method of claim 5, wherein a pH of the solution is 5 to 8.5.
